(19) Europäisches Patentamt
European Patent Office
Office européen des brevets

(11) Publication number: **0 148 314 A1**

(12) # EUROPEAN PATENT APPLICATION

(21) Application number: 84106230.0

(22) Date of filing: 30.05.84

(51) Int. Cl.⁴: C 07 C 17/08, C 07 C 21/04

(30) Priority: 12.12.83 US 560140

(43) Date of publication of application: 17.07.85 Bulletin 85/29

(84) Designated Contracting States: BE CH DE FR GB LI NL

(71) Applicant: **Union Camp Corporation, 1600 Valley Road, Wayne New Jersey 07470 (US)**

(72) Inventor: **McElligott, Lois T., 1158 Wheatsheaf Lane, Abington Pennsylvania 19001 (US)**

(74) Representative: **von Füner, Alexander, Dr. et al, Patentanwälte Schiff, von Füner Strehl, Schübel-Hopf, Ebbinghaus, Finck Mariahilfplatz 2 u. 3, D-8000 München 90 (DE)**

(54) **Hydrohalogenation of conjugated dienes in the presence of alcohols.**

(57) The disclosure is of an improved process for the hydrohalogenation of a conjugated diene in the presence of a catalyst, which comprises carrying out the hydrohalogenation in the present of an alcohol.

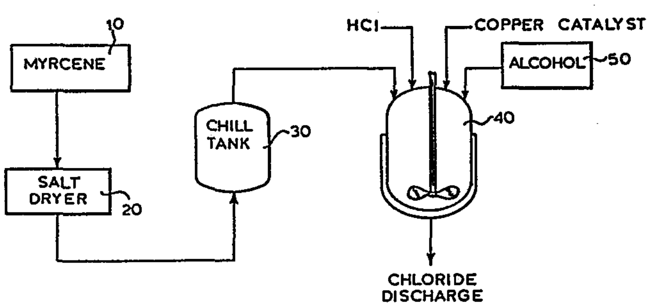


EP 0 148 314 A1

Union Camp Corporation

EPAA-31905.1
May 30, 1984

# HYDROHALOGENATION OF CONJUGATED DIENES IN THE PRESENCE OF ALCOHOLS

## BACKGROUND OF THE INVENTION

### Field of the Invention

The invention relates to processes for the hydrohalogenation of conjugated diene hydrocarbons

### Brief Description of the Prior Art

The literature is replete with descriptions of processes for the hydrohalogenation of conjugated dienes. Representative of such descriptions are those found in the U.S. Patents 2,882,323 and 3,016,408 and in British Patent 896,262;; see also German Offenlegungschrift 1,768,544. In the latter patent, hydrohalogenation of butadiene is conducted in the presence of aqueous mixtures including organic amines. The reaction mixtures are corrosive and in some reactions would tend to promote solvolysis. The German Auslegeschrift 1,253,264 describes the hydrochlorination of butadiene in a gas phase reaction. The present invention is an improvement over the prior art processes in that it may be carried out in liquid phases of the reactants, at low temperatures and with minimal solvolysis of products. Also, the process of the invention employs relatively inexpensive catalysts which are recoverable and reusable a plurality of times.

The present invention is particularly advantageous when used to hydrochlorinate myrcene. Myrcene is a conjugated diene of the formula:-

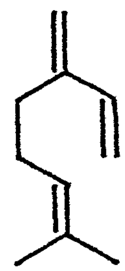

(I)

Upon un-catalyzed hydrochlorination of myrcene the major product is myrcenyl chloride. However, the commercially valuable products of myrcene hydrochlorination are the associated co-products, namely, geranyl chloride and neryl chloride. Hydrochlorination in the presence of a copper catalyst shifts the reaction in favor of the desired co-products. It has been postulated that the hydrochlorination of myrcene in the presence of a copper catalyst proceeds according to the reaction scheme:-

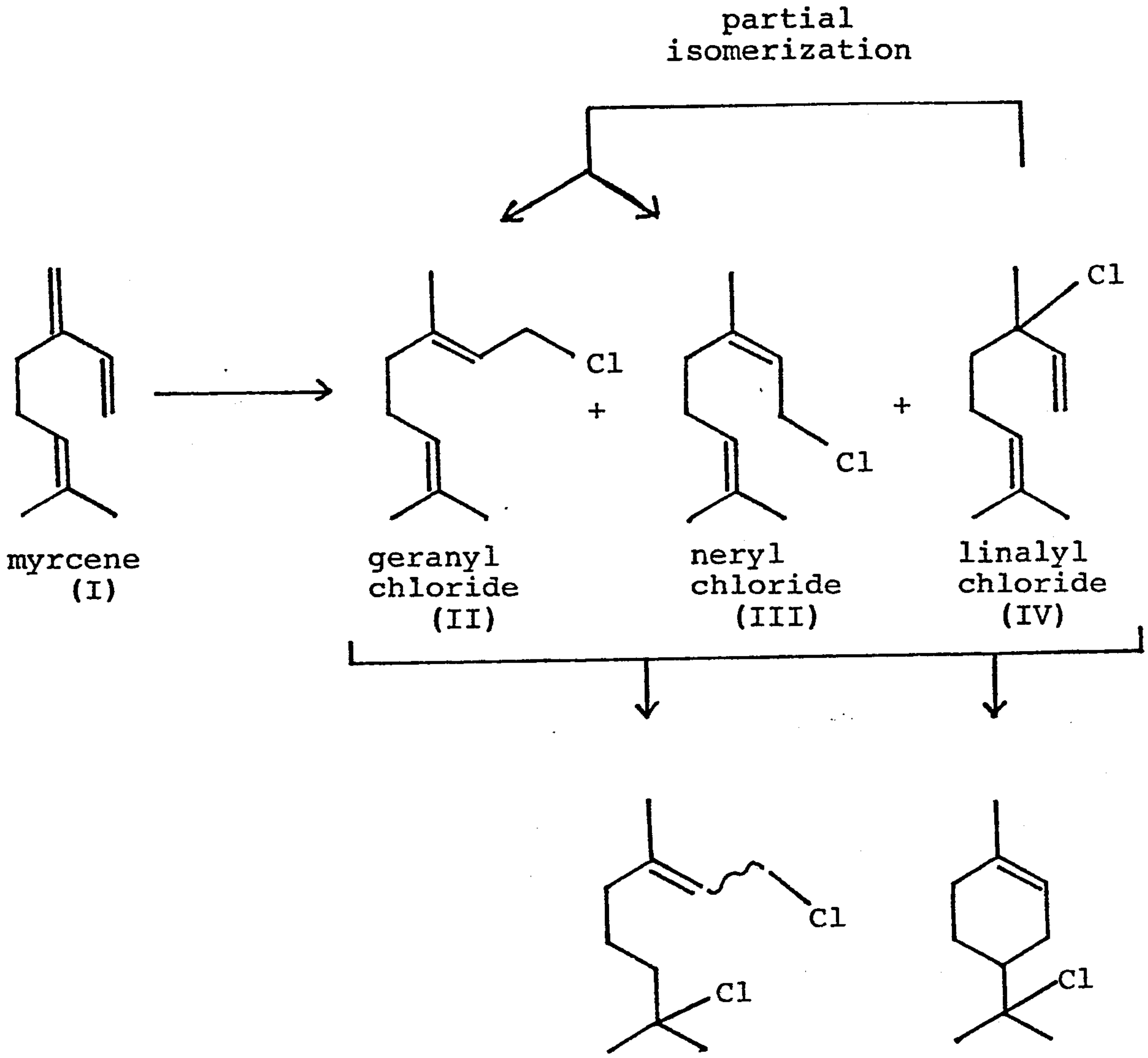


myrcene (I)

geranyl chloride (II) + neryl chloride (III) + linalyl chloride (IV)

dichlorides (V)

alpha-terpinyl chloride (VI)

Cyclization may also undesirably occur to form the alpha-terpinyl chlorides.

When the copper catalyst employed is in the form of cupric chloride ($CuCl_2$) the products generally include substantial proportions of linalyl chloride and lesser proportions of the desired geranyl and neryl chlorides. When the copper catalyst is in the form of cuprous chloride, the linalyl chloride product is lessened due, apparently, to partial isomerization to the desired geranyl and neryl chlorides.

From the above proposed reaction scheme, it will be appreciated that any process for hydrochlorination of myrcene, to be commercially feasible, must result in a favorable yield of the desired geranyl (II) and neryl (III) monochlorides and minimal formation on linalyl (IV) and alpha-terpinyl (VI) monochlorides. It was previously appreciated that the relative proportions of monochlorides (II), (III) and (IV) in the hydrochlorination product reaction mixture could be controlled to some degree by selection of the reaction temperature, gas flowrate and catalyst concentration.

We have now found that when the prior art hydrohalogenation of a conjugated diene, such as the hydrochlorination of isoprene and in particular the hydrochlorination of myrcene, is carried out in the presence of an alcohol, then the isomerization of the allylic chloride products during the hydrohalogenation reaction is shifted to favor formation of the less-substituted allylic

chloride, being geranyl chloride (II) and neryl chloride (III) in the above example.

SUMMARY OF THE INVENTION

The invention comprises, in a method for the hydrohalogenation of a conjugated diene which comprises, hydrohalogenating the diene under anhydrous, liquid phase conditions in the presence of a catalytic proportion of a hydrohalogenation catalyst, the improvement which comprises; adding to the hydrohalogenation reaction mixture a catalytic proportion of an alcohol.

The improved process of the invention may be carried out in a batch or a continous manner.

It is preferred to use as a diene myrcene or isoprene and then the halogen should be chlorine and the catalyst is a copper catalyst.

An improved process is carried out at a temperature from about -30°C to 50°C.

The preferred catalyst is cuprous catalyst and the preferred proportion of copper catalyst is within the weight range of 0.01 to 10.0 percent of dry diene.

The process is especially carried out for a period of from 3 to 15 hours.

It is furthermore preferred to use a molar ratio of alcohol to copper catalyst within the weight range of from 0.1 to 5.0.

The preferred alcohol is selected from the group of the formula:

$$R — OH$$

wherein R is selected from the group consisting of alkyl having 1 to 25 carbon atoms, inclusive and aralkyl having 7 to 25 carbon atoms, inclusive, and is especially selected from the group consisting of isobutanol, isopropanol, hexanol, cyclohexanol, 3-pentanol, octanol, decanol and geraniol.

## BRIEF DESCRIPTION OF THE DRAWING

The drawing is a schematic representation of a preferred embodiment method of the invention.

## DETAILED DESCRIPTION OF THE PREFERRED EMBODIMENTS OF THE INVENTION

The process of the invention may be employed for the hydrohalogenation of any conjugated diene. Representative of such dienes are myrcene, isoprene, 2-methylpiperylene, *beta*-phellandrene, 2-ethylbutadiene, ocimene, alloocimene,

1-phenylbutadiene, and the like.

The method of the invention is particularly advantageous for the hydrohalogenation of myrcene to obtain the geranyl and neryl halides which are intermediates for the manufacture of commercially valuable geraniol and nerol. When applied to myrcene, the improved method of the invention will increase the overall yield of the geranyl and neryl halides over the prior art processes and will improve selectivity of the ratio of geranyl isomer to the neryl isomer. Commercially available myrcene made by pyrolysis of beta-pinene, purified forms of myrcene, and myrcene isolated from natural materials may be provided as the starting material in the preferred process of the invention.

The accompanying drawing is a schematic representation of a preferred embodiment method of the invention for the hydrochlorination of myrcene. As shown in the Figure the provided myrcene initially held in tank 10 may be first dried in a conventional salt bed dryer 20 to remove water, as necessary. The dried myrcene is then preferably cooled to a temperature in the range of from about -30°C to about 30°C.; most preferably circa 10°C. in a cooling unit 30. Alternatively, the myrcene may be cooled first and then processed through a salt bed dryer 20 to remove water. Cooling the starting myrcene prior to drying in the salt bed dryer 20 is somewhat advantageous in that pre-cooling increases drying efficiency in the salt bed dryer 20.

As shown in the Figure the dried and cooled myrcene starting material may be passed into a hydrohalogenation apparatus which comprises in the preferred embodiment a stirred tank reactor 40. In the reactor 40, hydrohalogenation of the introduced myrcene is carried out in the presence of a catalytic proportion of a hydrohalogenation catalyst, at a temperature within the range of from about -30°C. to about 50°C.; preferably at a temperature within the range of from -10°C. to 25°C., most preferably about 10°C. Hydrohalogenation may be effected, for example, by reaction of the myrcene with a hydrogen halide like hydrogen chloride or hydrogen bromide, in substantially anhydrous form and under substantially anhydrous conditions, i.e. having less than about 5% water present in the reaction mixture. As shown in the figure, the preferred hydrohalogenation is with gaseous hydrogen chloride which is introduced as a gas possibly generated in a vaporizer, and then metered into the reactor 40, via appropriate conduits. Advantageously, the hydrogen chloride is metered into the reactor 40 at a rate of from about 2.0 to about 300 gms/hour/mole of myrcene present in the reactor 40. Preferably, the rate is from about 4.0 to 8.0 gms/hour/mole of myrcene.

The alcohol is introduced into the reactor 40 from storage vessel 50. Myrcene, alcohol, hydrogen chloride and copper catalyst are introduced into the reactor 40 sequentially. Preferably, the reactor 40 after purging with

an inert gas such as nitrogen is first charged with the cool myrcene, the copper catalyst, and the alcohol. While cooling and stirring, the hydrogen chloride is added incrementally to the charge.

A wide variety of catalysts for hydrohalogenation of myrcene are well known and include, for example, any copper compound having a valency of 2 or less, including metallic copper. Any copper compound convertible to the halide such as the bromide, iodide or chloride under conditions of the reaction may also be used. Representative of copper catalysts advantageously employed are the chloride, bromide, carbonate, oxide, acetate, formate, sulfate, and like derivative cupric and cuprous compounds. Preferred as the hydrochlorination catalyst in the improved process of the invention is cuprous chloride. Catalytic proportions of the anhydrous hydrohalogenation catalyst are generally within the weight range of from about 0.01 to 10 percent of the dry diene, preferably about 0.5 percent, especially myrcene.

A wide range of alcohols may be employed in the improved process of the invention. Advantageously the alcohol selected is one which is a fluid under the process conditions and inert to reaction with the reaction mixture ingredients. Representative of such alcohols are hexanol, decanol, geraniol, isopropanol, isobutanol, 3-pentanol, decanol, cyclohexanol, and the like. Preferred alcohols are those of the formula:-

$$R - OH \qquad (VII)$$

wherein R represents alkyl or aralkyl.

The term "alkyl" as used herein means the monovalent moiety obtained upon removal of a hydrogen atom from a parent alkane. Representative of alkyl are alkyl to 1 to 25 carbon atoms, inclusive, such as methyl, ethyl, propyl, butyl, pentyl, hexyl, heptyl, octyl, nonyl, undecyl, decyl, dodecyl, octadecyl, nonodecyl, eicosyl, heneicosyl, docosyl, tricosyl, tetracosyl, pentacosyl and the isomeric forms thereof. The term aralkyl as used herein means alkyl as defined above wherein a hydrogen atom has been replaced with a monovalent moiety obtained upon removal of a hydrogen atom from an aromatic hydrocarbon. Representative of aralkyl are aralkyl of 7 to 25 carbon atoms, inclusive, such as benzyl, phenethyl, phenpropyl, phenbutyl, phenhexyl, napthoctyl and the like.

Preferably, the R moiety will contain from 2 to 22 carbon atoms, inclusive; most preferably 6 to 10 carbon atoms, inclusive, The most preferred alcohols will not contain nitrogen containing groups which might degrade under reaction conditions.

It will be appreciated that under specific conditions of operating the process of the invention, certain of the above described alcohols of the formula (VII) given above have advantages over other compounds of the same general formula. Selection of a particular alcohol (VII) for use under specific process conditions, for optimum yields may be made by trial and error technique.

The alcohol is used in a proportion to isomerize, during the hydrohalogenation reaction, at least some of the more-substituted allylic chloride produced in the method of the invention. Such a proportion is generally within the range of from about 0.01 to 10 percent by weight of the diene charge, preferably 0.1 to 5 percent. This proportion of alcohol catalyst is added to the hydrohalogenation reaction mixture, without consideration of any alcohol by-product which may have been undesirably formed in the reaction mixture by solvolysis of desired product. Optimum proportions will depend to some extent upon the alcohol selected and may be determined by trial and error technique.

The controlling reaction rate in the hydrohalogenation process of the invention is the isomerization of the more-substituted halide to the desired less-substituted halide. This is controlled by residence time in the hydrohalogenation reaction zone. We have found that in hydrochlorination of myrcene, the preferred minimum total residence time is within the range of from 3 to 15 hours,

and most preferably 5 to 8 hours under the above described operating temperatures. The presence of linalyl chloride in the reaction mixture may be monitored by conventional analytical techniques. Longer residence times in the hydrochlorination reactor may cause a yield loss due to conversion of the monochlorides to <u>alpha</u>-terpinyl chloride. Shorter residence times may not be sufficient to isomerize the linalyl chloride to the desired geranyl/neryl chlorides.

When it has been determined that hydrohalogenation has occurred to a maximum desired point, the hydrohalogenation product mixture is passed from the hydrohalogenation apparatus. The desired hydrohalogenated diene may be separated from the reaction mixture employing conventional and known techniques including washing, decantation, distillation and like techniques. The alcohol may be recovered by distillation and reused a plurality of times.

The following examples describe the manner and process of making and using the invention and set forth the best mode contemplated by the inventors of carrying out the invention but are not to be construed as limiting. All parts given are by weight unless otherwise indicated.

<u>EXAMPLES 1-8</u>

To a 1 liter reaction vessel there is charged 300.0g myrcene (70 wt%), 1.0g cuprous chloride, and an alcohol as described in <u>Table</u> 1 below. The mixture is purged with

nitrogen and cooled to 0°C. Hydrogen chloride gas is added at a rate of 7-8 g/hour while maintaining the temperature at 10°C. At the end of reaction (7 hours), monitored by infrared spectroscopy to a 1% myrcene level, the reaction product is neutralized with sodium carbonate and aqueous sodium hydroxide. The product is analyzed by gas chromatography. The results are shown in Table 1. Linalyl, neryl and geranyl chlorides are abbreviated LCL, NCL, and GCL respectively in the Table 1.

TABLE 1

| Example No. | Alcohol and amount used | Molar Yield of Products (%) | | GCL:NCL Ratio |
|---|---|---|---|---|
| | | LCL | GCL+NCL | |
| 1 | None (control) | 18 | 69 | 1.26 |
| 2 | Isobutanol, 0.7g | 8 | 79 | 1.30 |
| 3 | Isopropanol, 0.6g | 7 | 79 | 1.34 |
| 4 | Hexanol, 1.0g | 10 | 76 | 1.23 |
| 5 | Cyclohexanol, 1.0g | 12 | 76 | 1.17 |
| 6 | 3-Pentanol, 0.9g | 9 | 76 | 1.24 |
| 7 | Geraniol, 1.5g | 10 | 73 | 1.21 |
| 8 | n-Decanol, 1.5g | 9 | 77 | 1.30 |

0148314

EXAMPLE 9

To a chilled 50 ml reaction vessel there is charged 10.0g isoprene, 0.1g cuprous chloride and isobutanol in an amount indicated in Table 2 below. The mixture is cooled to a temperature of 0°C. Hydrogen chloride gas is added at a rate of 1.5-2.0 g/hour while maintaining the temperature at 0°C.. At the end of the reaction (4 hours), monitored by weight, the reaction product is neutralized and analyzed by nuclear magnetic resonance spectroscopy to determine the amounts of 3-chloro-3-methyl-1-butene and 1-chloro-3-methyl-2-butene (abbreviated 3,3,1-CMB and 1,3,2-CMB respectively). The result of this analysis is shown in Table 2, below.

TABLE 2

| Amount Isobutanol used | Composition of Product (%) 3,3,1-CMB | 1,3,2-CMB |
|---|---|---|
| None (control) | 28 | 72 |
| 0.07g | 6 | 94 |

WHAT IS CLAIMED:

1. In a method for the hydrohalogenation of a conjugated diene which comprises hydrohalogenating the diene under anhydrous, liquid phase conditions in the presence of a catalytic proportion of a hydrohalogenation catalyst, the improvement which comprises; adding to the hydrohalogenation reaction mixture a catalytic proportion of an alcohol.

2. The improved method of claim 1 wherein the diene is myrcene, the halogen is chlorine and the catalyst is a copper catalyst.

3. The improved method of claim 1 wherein the diene is isoprene, the halogen is chlorine and the catalyst is a copper catalyst.

4. The improved process of one of the preceeding claims wherein the temperature of reaction is from about -30°C to 50°C.

5. The process of one of the preceeding claims wherein the catalyst is cuprous chloride.

6. The process of one of the preceeding claims wherein the catalytic proportion of copper catalyst is within the weight range of 0.01 to 10.0 percent of the dry diene.

7. The process of one of the preceeding claims wherein the hydrohalogenation is carried out for a period of from 3 to 15 hours.

8. The process of one of the preceeding claims wherein the molar ratio of alcohol to copper catalyst is within the range of from 0.1 to 5.0.

9. The process of one of the preceeding claims wherein the alcohol is selected fromthe group of the formula:

$$R — OH$$

wherein R is selected from the group consisting of alkyl having 1 to 25 carbon atoms, inclusive and aralkyl having 7 to 25 carbon atoms, inclusive.

10. The process of claim 9 wherein the alcohol is selected from the group consisting of isobutanol, isopropanol, hexanol, cyclohexanol, 3-pentanol, octanol, decanol and geraniol.

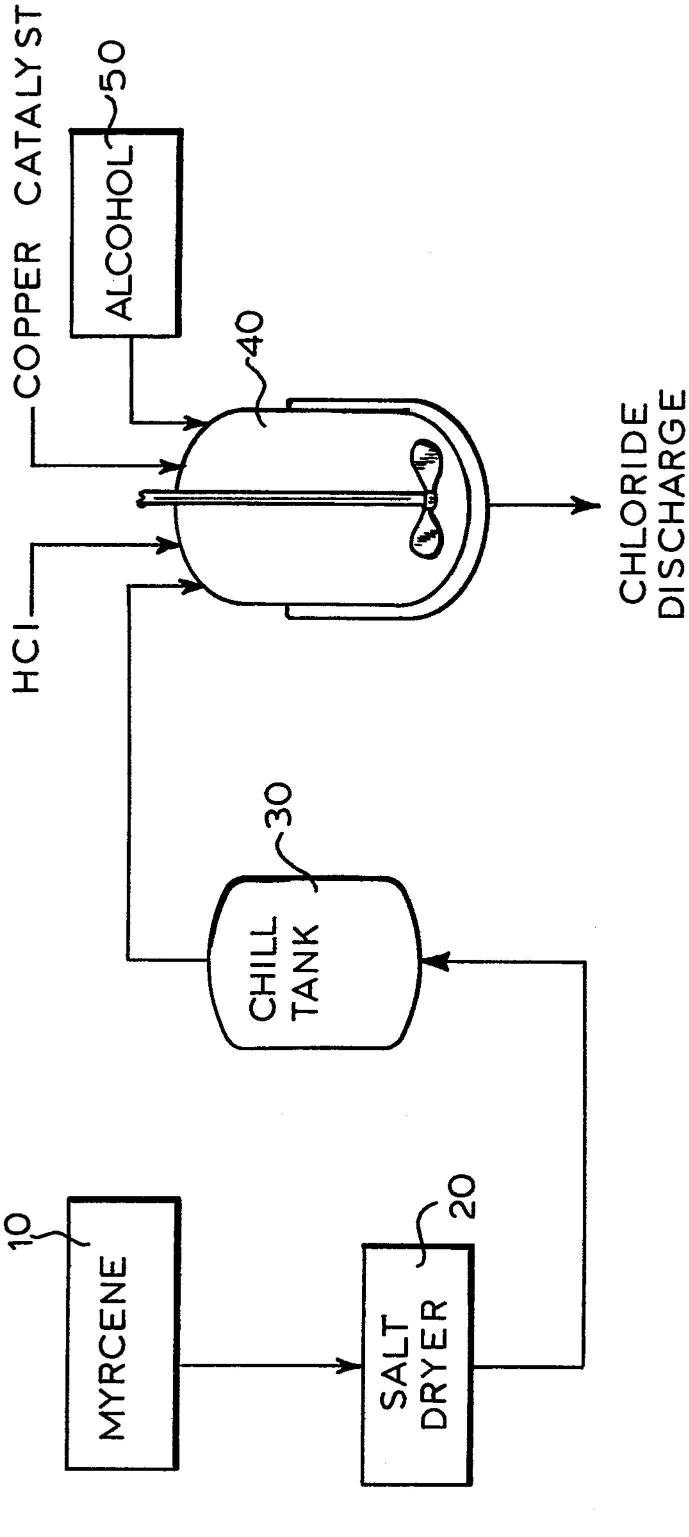
COPPER CATALYST
ALCOHOL
50
40
HCl
CHLORIDE DISCHARGE
CHILL TANK
30
MYRCENE
10
SALT DRYER
20

European Patent Office

# EUROPEAN SEARCH REPORT

0148314

Application number

EP 84 10 6230

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | DE-B-1 768 544 (CHEMISCHE WERKE HUELS)<br>* Claim 1 *<br>--- | 1,5 | C 07 C 17/08<br>C 07 C 21/04 |
| A | US-A-2 999 887 (J.B. FINLAY)<br>* Claim 1; column 15, line 8 *<br>--- | 1,5 | |
| A,D | US-A-2 882 323 (R. WEISS)<br>* Claim 1; example 3 *<br>----- | 1,2,5 | |
| | | | TECHNICAL FIELDS SEARCHED (Int. Cl.4) |
| | | | C 07 C 17/08<br>C 07 C 21/04 |

The present search report has been drawn up for all claims

| Place of search | Date of completion of the search | Examiner |
|---|---|---|
| BERLIN | 06-03-1985 | KNAACK M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82